# EUROPÄISCHE PATENTSCHRIFT

(11) **EP 1 761 768 B1**
(45) Veröffentlichungstag und Bekanntmachung des Hinweises auf die Patenterteilung: **08.05.2019**
(21) Anmeldenummer: 05753653.4
(22) Anmeldetag: 15.06.2005
(51) Int. Cl.: G01N 33/02, G01N 33/14, G01N 33/15

(54) **VERFAHREN ZUR ECHTHEITSBESTIMMUNG VON MATERIALIEN MIT SAUERSTOFF 18 ISOTOPISCH MARKIERTEN SUBSTANZEN**
METHOD FOR DETERMINING THE AUTHENTICITY OF MATERIALS BY MEANS OF SUBSTANCES WHICH ARE ISOTOPICALLY MARKED WITH OXYGEN 18
PROCEDE POUR DETERMINER L'AUTHENTICITE DE MATIERES AU MOYEN DE SUBSTANCES MARQUEES ISOTOPIQUEMENT AVEC DE L'OXYGENE 18

(30) Priorität: 30.06.2004 DE 102004031486
(43) Veröffentlichungstag der Anmeldung: 14.03.2007
(73) Patentinhaber: Forschungszentrum Jülich GmbH, 52425 Jülich (DE); Agroisolab GmbH, 52428 Jülich (DE)
(72) Erfinder: BONER, Markus, 52428 Jülich (DE); FÖRSTEL, Hilmar, 50825 Köln (DE)
(86) Internationale Anmeldenummer: PCT/DE2005/001060
(87) Internationale Veröffentlichungsnummer: WO 2006/002608

(56) Entgegenhaltungen:
- WO-A-03/014700
- DE-A1- 10 200 802
- US-A- 5 179 027
- US-A- 5 474 937
- US-A- 5 849 590
- US-A1- 2004 029 294
- ROSSMANN A ET AL: "Stable oxygen isotope content of water of EU data-bank wines from Italy, France and Germany" ZEITSCHRIFT FUER LEBENSMITTEL-UNTERSUCHUNG UND -FORSCHUNG. A, EUROPEAN FOOD RESEARCH AND TECHNOLOGY, SPRINGER, HEIDELBERG, DE, Bd. 208, Nr. 5-6, 1999, Seiten 400-407, XP002215533 ISSN: 1431-4630
- PUPIN A M ET AL: "USE OF ISOTOPIC ANALYSES TO DETERMINE THE AUTHENTICITY OF BRAZILIAN ORANGE JUICE (CITRUS SINENSIS)" JOURNAL OF AGRICULTURAL AND FOOD CHEMISTRY, AMERICAN CHEMICAL SOCIETY. WASHINGTON, US, Bd. 46, Nr. 4, April 1998 (1998-04), Seiten 1369-1373, XP001146617 ISSN: 0021-8561
- ROSSMANN A ET AL: "MULTIELEMENT STABLE ISOTOPE RATIO ANALYSIS OF GLYCEROL TO DETERMINEITS ORIGIN IN WINE" ZEITSCHRIFT FUER LEBENSMITTEL-UNTERSUCHUNG UND -FORSCHUNG. A, EUROPEAN FOOD RESEARCH AND TECHNOLOGY, SPRINGER, HEIDELBERG, DE, Bd. 207, Nr. 3, 1998, Seiten 237-243, XP000982669 ISSN: 1431-4630
- PATENT ABSTRACTS OF JAPAN Bd. 2003, Nr. 03, 5. Mai 2003 (2003-05-05) & JP 2002 329179 A (TOKYO GAS CO LTD), 15. November 2002 (2002-11-15)

## Beschreibung

Die Erfindung betrifft ein Verfahren zur Echtheitsbestimmung von Materialien nach dem Oberbegriff des Anspruchs 1 und 2.

Allgemeines Ziel ist es, Materialien, wie Futter- und Nahrungsmittel sowie die daraus entstandenen tierischen und pflanzlichen Erzeugnisse, aber auch produzierte Nahrungs- und Genussmittel, so zu kennzeichnen, dass es dem Hersteller oder Auftraggeber ermöglicht wird, zu überprüfen, ob es sich bei einem Produkt um das von ihm erzeugte Futter- oder Nahrungsmittel bzw. das von ihm hergestellte Produkt handelt.

Die deutsche Patentanmeldung der Anmelderin DE 102 00 802 A2 offenbart ein Markierungsverfahren bei dem beispielsweise Lebensmittel mit Wasser angereichert sind, welches mit Deuterium angereichert ist. Dies führt zu einer Isotopenkorrelation zwischen D/H zu ¹⁸O/¹⁶O, welche von der natürlichen Korrelation abweicht, so dass die Herkunft charakterisiert werden kann, wenn die vom Hersteller gewählte Isotopenkorrelation bekannt ist. Das Verfahren nach der DE 102 00 802 A2 ist vor allen Dingen für die Kennzeichnung von Flüssigkeiten, wie Getränken geeignet.

Es besteht daher der Bedarf, ein Verfahren zur Echtheitsbestimmung von Stoffen zur Verfügung zu stellen, welches eine Markierung eines weiteren Spektrums an Materialien ermöglicht. Insbesondere sollen Lebens- oder Futtermittel aber auch Arzneimittel oder Mittel für die Homöopathie oder Kosmetika in ihrer Herkunft oder Chargenzugehörigkeit gekennzeichnet werden können. Das Verfahren zur Kennzeichnung und die zur Kennzeichnung eingesetzten Mittel sollen ungiftig sein, lebensmittel und futtermittelrechtlichen Vorgaben genügen, vorzugsweise sollen die Mittel zur Kennzeichnung der Herkunft nicht als Fremdzusatz im rechtlichen Sinne gelten. Der Marker soll kostengünstig sein, da hohe Kosten insbesondere im Lebensmittel- und Futtermittelbereich nicht akzeptiert werden. Außerdem müssen die Verfahren der Herstellung der Markierung derart ausgelegt sein, dass möglichst wenig Material der teuren Markierungssubstanz verloren geht, d.h. es ist sogar ein vollständiger Umsatz der zur Produktion des Markers eingesetzten Markierung anzustreben. So ist beispielsweise aus der US 5474937 ein Verfahren zur Bestimmung der Echtheit von Materialien bekannt, bei dem dem Material eine Substanz zugesetzt wird, welche ¹⁸O in einer Konzentration enthält, welche über oder unter der in der Natur vorkommenden ¹⁸O Konzentration liegt, und die zur Echtheitsbestimmung wieder insoliert wird. Allerdings wird hier nicht offenbart, wie eine Rückgewinnung der Markersubstanz möglich ist.

Ausgehend vom Oberbegriff des Anspruchs 1 sowie dem nebengeordneten Anspruch 2 wird die Aufgabe erfindungsgemäß dadurch gelöst, dass die Materialien, deren Echtheit zu kennzeichnen ist, mit mindestens einer Substanz markiert sind, die das natürlich vorkommende Sauerstoffisotop ¹⁸O in einer Anreicherungsmenge oder Abreicherung enthält, die größer bzw. kleiner ist als diejenige Konzentration, die innerhalb des natürlichen Schwankungsfensters in der Natur vorkommt.

So wird die Aufgabe erfindungsgemäß dadurch gelöst, dass dem Material mindestens eine Substanz, welche ¹⁸O in einer Konzentration enthält, welche über oder unter der in der Natur vorkommenden ¹⁸O Konzentration von 0,1885 bis 0,2126 Atomgewichtsprozent ¹⁸O liegt, beigefügt wird und zur Echtheitsbestimmung wieder isoliert wird, indem sie durch Veraschung freigesetzt wird, wobei die Substanz bis zu 1000°C thermisch stabil ist, die ¹⁸O Konzentration der Substanz bestimmt wird und anschließend analysiert wird, ob deren ¹⁸O Konzentration oberhalb oder unterhalb der natürlich vorkommenden ¹⁸O Konzentration liegt oder anschließend die ¹⁸O Konzentration der Substanz mit der ¹⁸O Konzentration verglichen wird, die die Substanz hat, welche dem Material zur Markierung beigefügt wurde.

Mit dem erfindungsgemäßen Verfahren und den Markerverbindungen ist es nunmehr möglich Materialien im Hinblick auf ihre Echtheit zu kennzeichnen.

Unter Nachweis der Echtheit im Sinne der Erfindung können alle möglichen Anwendungen fallen. So können die Herkunft bezüglich eines Herstellers oder Zwischenlieferanten, die Herkunft bezüglich der geographischen Lage, die Chargenzugehörigkeit, das Herstellverfahren, die Verwendung, Weitergabe oder Verkauf, der tatsächliche Einsatz gemäß vertraglicher Regelung oder ähnliches gemeint sein.

Vorteilhafte Weiterbildungen sind in den Unteransprüchen angegeben.

Im Folgenden soll die Erfindung allgemein erläutert werden.

Erfindungsgemäß wird dem Material, dessen Herkunft zu kennzeichnen ist beziehungsweise deren Echtheit zu bestimmen ist, eine Verbindung zugesetzt, welche das Sauerstoffisotop ¹⁸O in einer Konzentration enthält, welche von der maximalen in der Natur vorkommenden Konzentration nach oberen Werten oder von der minimal in der Natur vorkommenden Konzentration nach unteren Werten abweicht.

Die Abweichung ist dabei vorzugsweise mindestens so groß, wie der Messfehler zur Bestimmung der Konzentration an ¹⁸O in der Substanz.

Bevorzugt ist die Markersubstanz mit ¹⁸O hochangereichert. Unter hochangereichert ist ein Bereich zu verstehen, der oberhalb der natürlichen ¹⁸O Konzentration liegt. In der Natur haben alle Sauerstoff enthaltenden Stoffe, innerhalb eines bekannten Schwankungsfensters, in etwa die gleiche ¹⁸O Konzentration. Angereichert im Sinne der Erfindung bedeutet daher, dass die ¹⁸O Konzentration nach oberen Werten außerhalb dieses Schwankungsfensters angesiedelt ist.

Das natürliche Schwankungsfenster liegt in einem Bereich von 0,1885 bis 0,2126 Atomgewichtsprozent ¹⁸O.

Besonders bevorzugt ist der Sauerstoff in der Markersubstanz wenigstens annähernd zu 100% durch ¹⁸O ersetzt.

Die Erfindung ist aber auch dann durchführbar, wenn die ¹⁸O Konzentration in der Markersubstanz 0,4, 1, 2, 3, 4, 5, 10, 20, 30, 40, 50, 60, 70, 80, 90, 95, oder 99 Atomgewichts- % Sauerstoff beträgt.

In einer alternativen Ausführungsform ist die Markersubstanz an ¹⁸O abgereichert. Unter abgereichert ist ein Bereich zu verstehen, der unterhalb der natürlichen ¹⁸O Konzentration liegt. Abgereichert im Sinne der Erfindung bedeutet daher, dass die ¹⁸O Konzentration nach unteren Werten außerhalb dieses Schwankungsfensters angesiedelt ist.

Die Erfindung ist auch durchführbar, wenn die ¹⁸O Konzentration in der Markersubstanz 0,01 Atomgewichts-% Sauerstoff beträgt.

Vorzugsweise soll die Konzentration der ¹⁸O-haltigen Verbindung in dem zu markierenden Material so hoch sein, dass eine Verdünnung der Materialprobe, in der sich die Substanz befindet, auf 1/10 noch einen genauen Herkunftsnachweis liefert.

Die Markierungssubstanzen können in geringen Konzentrationen eingesetzt werden, wie beispielsweise 50mg/t zu markierendem Material. Dies macht die Anwendung kostengünstig und führt zu lebensmittel- und futtermittelrechtlich unbedenklichen Mengen der Markerstoffe in den Materialien.

Die zugesetzte Substanz kann chemisch (bis auf die Isotopenzusammensetzung) ein Stoff sein, welcher natürlicher Bestandteil des Materials ist, welches er im Hinblick auf die Herkunft kennzeichnen soll, es kann jedoch auch ein Stoff sein, welcher kein natürlicher Bestandteil des Materials ist.

Bei der Markerverbindung kann es sich um eine Flüssigkeit, um einen Feststoff, um einen in Wasser löslichen Stoff oder um einen in Wasser unlöslichen Stoff handeln.

Vorzugsweise sollte die Substanz im Hinblick auf das Material, welchem sie zugesetzt wird, chemisch inert sein, das heißt nicht mit ihm reagieren.

Vorzugsweise sollte die mit ¹⁸O-markierte Substanz Säure- und/oder Base stabil sein, das heißt sich zumindest in der Material nicht unter Einfluss von Säure und/oder Base zersetzen oder sonst reagieren.

Weiterhin muss die ¹⁸O-haltige Markierungssubstanz hitzebeständig sein, das heißt sie sollte sich zumindest unter normalen Bedingungen, denen Lebens- und Futtermittel sowie Kosmetika, Arzneimittel oder Homöopathiemittel unterliegen, nicht thermisch zersetzt werden. Die Anforderungen an die thermische Stabilität hängen vom jeweiligen Material ab, dem die Markersubstanz zugeführt wird. Die Markersubstanz wird gemäss der Erfindung durch Veraschung freigesetzt, wie beispielsweise Eisenoxid, und muss bis zu 1000°C thermisch stabil sein.

Als Markierungsmittel kommen beispielsweise Eisenoxide, hochgeglühte Eisenoxide, wie Fe₂O₃, FeO, SiO₂, Silikate oder Alumosilikate zum Einsatz, bei denen der Sauerstoff in einem Prozentsatz durch ¹⁸O ersetzt ist, welcher über oder unter dem natürlichen Prozentsatz liegt. Mittels IRMS und ggf. anderer geeigneter Methoden können angereicherte, abgereicherte bzw. isotop veränderte Zusätze trotz hoher Verdünnung nachgewiesen werden.

Zu markierende Materialien können Lebensmittel, Getränke, Futtermittel, Kosmetika, Homöopathika oder Arzneimittel sein. Es kann aber auch jedes Material markiert werden, in welches der Marker unter gemischt oder eingebunden werden kann. Für den Fall, dass Materialien markiert werden sollen, die nicht dem Verzehr oder der Einnahme in irgend einer Art zugeführt werden, brauchen die Marker nicht den lebensmittel- und futtermittelrechtlichen Vorgaben oder arzneimittelrechtlichen Vorgaben zu genügen.

Nach dem erfindungsgemäßen Verfahren wird die Markersubstanz mit einem bekannten prozentualen Anteil an ¹⁸O der Markierenden Substanz zugegeben. Das heißt das Isotopenverhältnis von ¹⁶O/¹⁸O ist bekannt. Soll die Herkunft bestimmt werden, so werden die Markersubstanzen isoliert bzw. abgetrennt und werden einem Analyseverfahren zugeführt, welches geeignet ist, die Isotopenverhältnisse zwischen ¹⁶O und ¹⁸O oder die absolute Menge an ¹⁸O in der Probe pro Gewichtseinheit isoliertem Marker zu bestimmen.

Als Bestimmungsmethoden für ¹⁸O können beispielsweise IRMS (isotope ratio mass spectrometry), ICP-MS (Induced cuppled plasma-MS) oder Mulitkollektor-ICP-MS eingesetzt werden.

Es besteht zur Echtheitsbestimmung die Möglichkeit, das Isotopenverhältnis zwischen dem natürlichen Vorkommen und dem tatsächlich gemessenen Wert des Stabilisotops als Echtheits- bzw. Herkunftsgröße zu verwenden oder aber auch nur die absolute Konzentration an Stabilisotop in dem markierten Material zu messen, um dessen Herkunft oder Echtheit zu bestimmen.

### Beispiele:

1. Eisen-II-Chlorid wird mit konzentrierter, ¹⁸O-angereicherter Natronlauge in Kontakt gebracht. Es entsteht angereichertes Eisenoxid. Wird bei der Veraschung von biologischem Material auch Eisenoxid aus vorhandenem Eisen gebildet, so ist diese Anreicherung trotzdem nachweisbar. Das Produkt wird durch Auswaschen vom Natriumchlorid abgetrennt und danach das Wasser durch Erhitzen abgetrennt. Durch weiteres Erhitzen bei höheren Temperaturen erhält man ein festes Magnetit, das sich einmal durch seine magnetischen Eigenschaften auszeichnet, aber auch durch seine isotope Markierung nachgewiesen werden kann. Es ist im Futter inert und damit selbst im Kot noch nachweisbar. Wird es einem Futtermittel zugesetzt, so ist es dort und im Tier nachweisbar. Der Sauerstoff ist durch Hochtemperaturpyrolyse und IRMS nachweisbar.
2. Siliciumtetrachlorid ergibt mit Wasser Siliciumdioxid (Sand). Ist das Wasser mit ¹⁸O angereichert, so ist es auch das physiologisch inerte Siliciumdioxid. Das kann gepulvert dem Futter zugesetzt werden.
3. Sulfurylchlorid reagiert mit Wasser zu Sulfat, das bei Einsatz von ¹⁸O/¹⁶O-markierten Wasser ebenfalls markiert ist. Mit Calcium gefällt, erhält man eine kristalline Verbindung, die als Markierung eingesetzt werden kann. Mit Ammoniak ist die Darstellung von Ammoniumsulfat möglich, einer gut wasserlöslichen Verbindung.

Bei der Markierung für die Echtheitsprüfung kann grundsätzlich folgendermaßen vorgegangen werden:
Eine Markersubstanz, enthaltend ¹⁸O einer definierten, bekannten Konzentration, wird dem zu markierenden Material zugegeben. Falls nötig, wird für eine homogene Durchmischung gesorgt. Soll die Echtheit des Materials geprüft werden, so wird die Markersubstanz isoliert, ggf. gereinigt und deren Konzentration an ¹⁸O bestimmt.

Ist die Markersubstanz für die markierte Probe ein Fremdstoff, so reicht es aus, entweder zu prüfen, ob der ¹⁸O Gehalt über oder unter dem natürlichen vorkommenden Gehalt an ¹⁸O in der Natur liegt oder quantitativ zu bestimmen, wie hoch die Konzentration an ¹⁸O in der Markersubstanz ist, um zu vergleichen, ob sie der eingesetzten Konzentration z. B. des Herstellers entspricht.

Enthält das zu markierende Material die Markersubstanz nach ihrer chemischen Zusammensetzung als natürlichen Bestandteil, so muss die Substanz isoliert werden und der natürlich vorkommende Anteil, der dem natürlich vorhandenen Isotopenverhältnis entspricht, berücksichtigt werden. Für den Fall, dass die Markersubstanz für die Probe keine Fremdsubstanz ist, so sollte die quantitative Zusammensetzung vom Marker und dem originären Gehalt der Substanz in der Probe bestimmt werden und daraus die ¹⁸O Atomgewichtsprozent des Markers berechnet werden.

Danach kann das Isotop mittels ICP-MS, am besten mit Multikollektor-ICP-MS, nachgewiesen werden. Die Isotope sind im Handel zu erwerben.

Die erfindungsgemäße Markierung kann auch bei der Kontrolle des Artenschutzes, beispielsweise Zucht und Handel exotischer Vögel eingesetzt werden, indem Futter bestimmter bekannter isotoper Zusammensetzung verwendet werden, die eine Herkunftsunterscheidung ermöglichen.

Insbesondere können folgende Güter im Hinblick auf ihre Echtheit bzw. Herkunft markiert werden.

Futtermittel, Futtermittelzusätze, Lebensmittel, Lebensmittelzusätze, Getränke, Getränkezusätze, Chemikalien, Aromastoffe, Düngemittel, Arzneimittel, Kosmetika, Homöopathika, vorzugsweise sofern ein Zusatz von Dispersionen oder Feststoffen möglich ist.

## Patentansprüche

1. Verfahren zur Bestimmung der Echtheit von Materialien,
**dadurch gekennzeichnet,**
**dass** dem Material mindestens eine Substanz, welche ¹⁸O in einer Konzentration enthält, welche über oder unter der in der Natur vorkommenden ¹⁸O Konzentration von 0,1885 bis 0,2126 Atomgewichtsprozent ¹⁸O liegt, beigefügt wird und zur Echtheitsbestimmung wieder isoliert wird, indem sie durch Veraschung freigesetzt wird, wobei die Substanz bis zu 1000°C thermisch stabil ist, die ¹⁸O Konzentration der Substanz bestimmt wird und anschließend analysiert wird, ob deren ¹⁸O Konzentration oberhalb oder unterhalb der natürlich vorkommenden ¹⁸O Konzentration liegt.

2. Verfahren zur Bestimmung der Echtheit von Materialien,
**dadurch gekennzeichnet,**
**dass** dem Material mindestens eine Substanz, welche ¹⁸O in einer Konzentration enthält, welche über oder unter der in der Natur vorkommenden ¹⁸O Konzentration von 0,1885 bis 0,2126 Atomgewichtsprozent ¹⁸O liegt, beigefügt wird und zur Echtheitsbestimmung wieder isoliert wird, indem sie durch Veraschung freigesetzt wird, wobei die Substanz bis zu 1000°C thermisch stabil ist, die ¹⁸O Konzentration der Substanz bestimmt wird und anschließend die ¹⁸O Konzentration der Substanz mit der ¹⁸O Konzentration verglichen wird, die die Substanz hat, welche dem Material zur Markierung beigefügt wurde.

3. Verfahren nach Anspruch 2,
**dadurch gekennzeichnet,**
**dass** die beigefügte Menge an Markersubstanz für den Fall, dass es sich bei dem Marker um keine Fremdsubstanz handelt, auf die Gesamtmenge der Substanz in der Probe normiert wird.

4. Verfahren nach einem der Ansprüche 1 bis 3,
**dadurch gekennzeichnet,**
**dass** eine chemisch inerte Substanz als Markersubstanz verwendet wird.

5. Verfahren nach Anspruch 4,
**dadurch gekennzeichnet,**
**dass** die Substanz gegenüber Säuren und/oder Basen stabil ist.

6. Verfahren nach einem der Ansprüche 1 bis 5,
**dadurch gekennzeichnet,**
**dass** die Markersubstanz mindestens eine Komponente aus der Gruppe bestehend aus hoch geglühtem Eisenoxid, Fe₂O₃, FeO, SiO₂, Silikate und Alumosilikate ist.

7. Verfahren nach einem der Ansprüche 1 bis 6,
**dadurch gekennzeichnet,**
**dass** das zu markierende Material mindestens eine Komponente aus der Gruppe bestehend aus Futtermittel, Futtermittelzusätze, Lebensmittel, Lebensmittelzusätze, Getränke, Getränkezusätze, Chemikalien, Aromastoffe, Düngemittel, Kosmetika, Homöopathika und Arzneimittel ist.

8. Verfahren nach einem der Ansprüche 1 bis 7,
**dadurch gekennzeichnet,**
**dass** es zur Kontrolle des Artenschutzes eingesetzt wird.

9. Verfahren nach Anspruch 8,
**dadurch gekennzeichnet,**
**dass** es für die Zucht und/oder für den Handel von Vögeln, insbesondere exotischen Vögeln verwendet wird.

## Claims

1. Method for determining the authenticity of materials, **characterised in that**
at least one substance, which contains ¹⁸O in a concentration that is above or below the naturally occurring ¹⁸O concentration of 0.1885 to 0.2126 atomic weight percent ¹⁸O, is added to the material and isolated again for determining authenticity by being released by incineration, in which the substance is thermally stable to 1000°C, the ¹⁸O concentration of the substance is determined and then it is analysed whether its ¹⁸O concentration is above or below the naturally occurring ¹⁸O concentration.

2. Method for determining the authenticity of materials, **characterised in that**
at least one substance, which contains ¹⁸O in a concentration that is above or below the naturally occurring ¹⁸O concentration of 0.1885 to 0.2126 atomic weight percent ¹⁸O, is added to the material and isolated again for determining authenticity by being released by incineration, in which the substance is thermally stable to 1000°C, the ¹⁸O concentration of the substance is determined and then the ¹⁸O concentration of the substance is compared with the ¹⁸O concentration, which the substance that was added to the material for marking has.

3. Method according to claim 2,
**characterised in that**
the amount of marker substance added, if the marker is not a foreign substance, is standardised at the total amount of the substance in the sample.

4. Method according to one of claims 1 to 3,
**characterised in that**
a chemically inert substance is used as the marker substance.

5. Method according to claim 4,
**characterised in that**
the substance is stable with regard to acids and/or bases.

6. Method according to one of claims 1 to 5,
**characterised in that**
the marker substance is at least a component from the group consisting of highly annealed iron oxide, Fe₂O₃, FeO, SiO₂, silicates and aluminosilicates.

7. Method according to one of claims 1 to 6,
**characterised in that**
the material to be marked is at least a component from the group consisting of feed, feed additives, food, food additives, drinks, drinks additives, chemicals, flavourings, fertilizers, cosmetics, homeopathic medicines and drugs.

8. Method according to one of claims 1 to 7,
**characterised in that**
it is used to monitor species protection.

9. Method according to claim 8,
**characterised in that**
it is used for breeding and/or trading in birds, particularly exotic birds.

## Revendications

1. Procédé de détermination de l'authenticité de matières,
**caractérisé**
**en ce que** l'on ajoute à la matière au moins une substance, qui contient O¹⁸ en une concentration au-dessus ou en dessous de la concentration d'O¹⁸ qui existe dans la nature, de 0,1885 à 0,2126 pour cent d'O¹⁸ en poids atomique, et, pour la détermination de l'authenticité, on l'isole à nouveau en la libérant par incinération, la substance étant stable thermiquement jusqu'à 1000°C, on détermine la concentration de O¹⁸ de la substance et on l'analyse ensuite sur le point de savoir si sa concentration d'O¹⁸ est au-dessus ou en dessous de la concentration d'O¹⁸ qui existe dans la nature.

2. Procédé de détermination de l'authenticité de matières,
**caractérisé**
**en ce que** l'on ajoute à la matière au moins une substance, qui contient O¹⁸ en une concentration au-dessus ou en dessous de la concentration d'O¹⁸ qui existe dans la nature, de 0,1885 à 0,2126 pour cent d'O¹⁸ en poids atomique, et, pour la détermination de l'authenticité, on l'isole à nouveau en la libérant par incinération, la substance étant stable thermiquement jusqu'à 1000°C, on détermine la concentration d'O¹⁸ de la substance et ensuite, on compare la concentration d'O¹⁸ de la substance à la concentration d'O¹⁸ qu'a la substance, qui a été ajoutée à la matière pour le marquage.

3. Procédé suivant la revendication 2,
**caractérisé**
**en ce que** l'on norme, sur la quantité totale de la substance dans l'échantillon, la quantité ajoutée de substance de marqueur, dans le cas où le marqueur n'est pas une substance étrangère.

4. Procédé suivant l'une des revendications 1 à 3,
**caractérisé**
**en ce que** l'on utilise, comme substance de marqueur, une substance inerte chimiquement.

5. Procédé suivant la revendication 4,
**caractérisé**
**en ce que** la substance est stable vis-à-vis des acides et/ou des bases.

6. Procédé suivant l'une des revendications 1 à 5,
**caractérisé**
**en ce que** la substance de marqueur est un constituant du groupe constitué d'oxyde de fer calciné à mort, Fe₂O₃, FeO, SiO₂, silicates et aluminosilicates.

7. Procédé suivant l'une des revendications 1 à 6,
**caractérisé**
**en ce que** la matière à marquer est au moins un constituant du groupe constitué d'aliments pour des animaux, d'additifs aux aliments pour des animaux, de denrées alimentaires, d'additifs de denrées alimentaires, de boissons, d'additifs de boissons, de produits chimiques, de substances aromatiques, d'engrais, de produits cosmétiques, de produits homéopathiques et de médicaments.

8. Procédé suivant l'une des revendications 1 à 7,
**caractérisé**
**en ce qu'**on l'utilise pour le contrôle de la protection des espèces.

9. Procédé suivant la revendication 8,
**caractérisé**
**en ce qu'**on l'utilise pour l'élevage et/ou pour le traitement d'oiseaux, notamment d'oiseaux exotiques.
